# DEMANDE DE BREVET EUROPEEN

(11) **EP 0 953 321 A1**
(43) Date de publication de la demande: **03.11.1999**
(21) Numéro de dépôt: 99400701.1
(22) Date de dépôt: 22.03.1999
(51) Int. Cl.: A61F 2/40

(54) **Prothèse de reconstruction d'épaule**

(30) Priorité: 28.04.1998 FR 9805328
(71) Demandeur: Depuy France, 69100 Villeurbanne (FR)
(72) Inventeur: Lesur, Etienne, 54220 Bayon (FR)
(74) Mandataire: Martin, Jean-Paul

(57) **Abrégé**

Cette prothèse de reconstruction d'épaule comporte une tête humérale sphérique (1) adaptée pour pouvoir être implantée dans la cavité glénoïde (2) d'une omoplate (3), une queue humérale (4) destinée à être introduite dans le canal médullaire d'un humérus, ainsi qu'un élément de connexion (5) entre la tête et la queue ; une fente (9 ou 17) est agencée dans l'une des deux surfaces (8, 16) en vis-à-vis de la tête et de la queue, la prothèse est pourvue d'un plot (5 ) solidaire de celle des ceux pièces qui ne présente pas la fente, dans laquelle le plot est adapté pour s'y engager, et il est prévu des moyens (14,15, 18, 19) pour régler la position longitudinale du plot dans la fente (9) et donc la position longitudinale de la tête par rapport à la queue ; de préférence la prothèse présente deux fentes (9, 17), orthogonales quand la prothèse est assemblée et le plot (5) est formé de deux demi-plots (11, 12) fixés l'un à l'autre et qui viennent s'engager dans une fente correspondante (9, 17); la position du plot (5) dans les fentes est réglable de sorte que la position de la tête (1) est réglable dans deux directions perpendiculaires, ce qui confère à la prothèse une grande adaptabilité à la glène (2).

## Description

La présente invention a pour objet une prothèse de reconstruction d'épaule, du type comportant une tête humérale sphérique, adaptée pour pouvoir être implantée dans la cavité glénoïde d'une omoplate, une queue humérale destinée à être introduite dans le canal médullaire d'un humérus, ainsi qu'un élément de connexion entre la tête et la queue.

Selon la technique antérieure connue, on assure la liaison entre la tête sphérique et la tige (queue) humérale en disposant des pièces intermédiaires multiples, qui constituent des systèmes relativement complexes et ne permettent pas en outre des réglages suffisamment satisfaisants. En particulier ces prothèses ne permettent pas d'orienter la tête dans toutes les directions. Les brevets EP 0 715 836 et EP 0 549 480 décrivent de telles prothèses d'épaule.

On connaît aussi d'autres réalisations, telles que celle enseignée par le brevet européen EP 299 889, qui nécessitent un aménagement particulier de la cavité glénoïde naturelle, et aussi des jeux de prothèses de dimensions différentes tels que décrits par la demande de brevet internationale W097/25943.

L'invention a pour but de réaliser une prothèse de reconstruction d'épaule, agencée de manière à pouvoir reconstruire l'anatomie pratiquement sans incidence sur les muscles et l'omoplate, ou avec une incidence minimale et contrôlée, afin de s'adapter au mieux à l'anatomie sans affecter celle-ci.

Conformément à l'invention, la prothèse de reconstruction d'épaule est caractérisée en ce qu'une fente est agencée dans l'une des deux surfaces en vis-à-vis de la tête et de la queue, en ce que la prothèse est pourvue d'un plot solidaire de celle des deux pièces qui ne présente pas la fente, dans laquelle le plot est adapté pour s'y engager, et en ce qu'il est prévu des moyens pour régler la position longitudinale du plot dans la fente et donc la position longitudinale de la tête par rapport à la queue.

La fente peut donc être réalisée soit dans la surface de la partie proximale de la queue, soit dans la face de la tête sphérique ou plus exactement de la calotte sphérique constituant la tête; le plot de liaison est solidarisé de l'autre pièce par tout moyen adéquat, et engagé de manière réglable dans la fente, afin de permettre un réglage de la position de la tête en vue d'une bonne adaptation à l'anatomie de la glêne.

Suivant un mode de réalisation avantageux, la prothèse présente deux fentes, sensiblement orthogonales quand la tête et la queue sont assemblées, le plot est formé de deux demi-plots fixés l'un à l'autre bout à bout et qui peuvent respectivement s'engager dans une fente correspondante; cet engagement respectif permet un réglage de la tête par rapport à la queue dans les deux directions orthogonales définies par les fentes.

On comprend que grâce à cette double possibilité de réglage de la position de la tête dans deux directions orthogonales, la tête peut être placée au mieux pour s'adapter à l'anatomie de la cavité glénoïde naturelle, sans que le chirurgien ait besoin de porter atteinte à celle-ci.

Suivant une autre particularité de l'invention, les faces opposées du plot venant respectivement en regard du fond de la fente de la queue et en regard du fond de la fente de la tête sont planes, et soit inclinées l'une par rapport à l'autre, soit parallèles entre elles.

Dans le cas où ces fentes présentent une inclinaison relative, cette inclinaison est réalisée soit en antéversion, soit en rétroversion d'un angle approprié, par exemple 6 degrés environ. Elle peut également être utilisée pour s'adapter à l'angle souhaité de varisation ou de valgisation de la tête humérale. On obtient ainsi une possibilité supplémentaire de réglage fin de la position de la tête de la prothèse.

D'autres particularités et avantages de l'invention apparaîtront au cours de la description qui va suivre, faite en référence aux dessins annexés qui en illustrent deux formes de réalisation à titre d'exemples non limitatifs.

La figure 1 est une vue en perspective éclatée, à l'échelle, d'une première forme de réalisation de la prothèse de reconstruction d'épaule selon l'invention ainsi que d'une omoplate associée d'un patient, cette omoplate étant vue dans le plan frontal.

La figure 2 est une vue de dessus du plot de la prothèse de la figure 1.

La figure 3 est une vue en élévation à échelle agrandie du plot des figures 1 et 2.

La figure 4 est une vue mi-coupe partielle mi-élévation, à échelle agrandie, de la partie proximale de la queue et de la tête de la prothèse d'épaule visée par l'invention, équipée d'un plot de liaison selon une seconde forme de réalisation de l'invention.

La figure 5 est une vue analogue à la figure 4 montrant une disposition différente du plot de liaison permettant d'orienter différemment la tête sphérique.

La figure 6 est une vue en élévation sensiblement à l'échelle, de la prothèse à assembler au moyen du plot de liaison des figures 4 et 5.

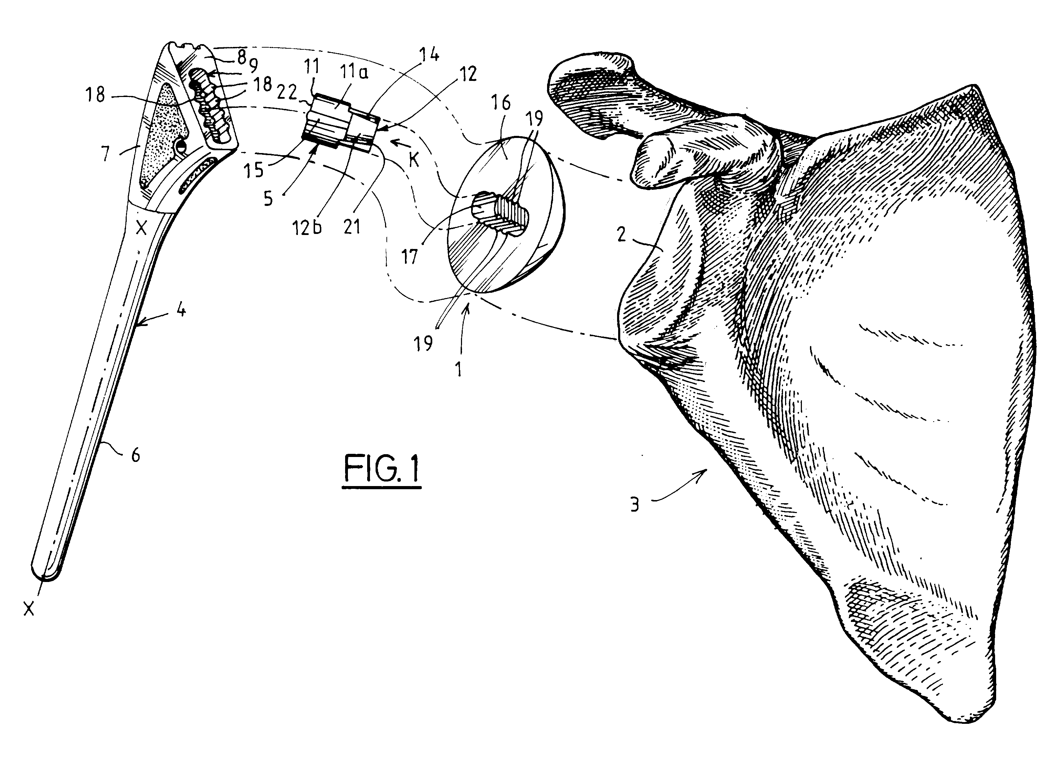

La prothèse de reconstruction d'épaule illustrée aux figures 1 à 3 comporte une tête 1 constituée d'une calotte sphérique destinée à venir se positionner dans la cavité glénoïde 2 d'une omoplate 3 d'un patient, une tige ou queue humérale 4 destinée à être introduite dans le canal médullaire d'un humérus non représenté, ainsi qu'un élément 5 de connexion entre la tête 1 et la queue 4.

Cette dernière est constituée classiquement d'une tige 6 d'axe longitudinal XX, prolongée, à sa partie proximale, proche de la tête humérale 1, par une partie évasée et incurvée en fonction du débouché qui sera pratiqué dans le canal médullaire de la partie supérieure de l'humérus, grâce à une râpe de forme correspondante. La queue 4 présente, à son extrémité proximale une surface 8 inclinée sur l'axe XX, et l'axe YY de la partie proximale et de la tête 1 est incliné sur l'axe XX d'un angle B, d'environ 130 degrés.

Dans cette surface 8 est ménagée une fente longitudinale 9 adaptée pour y permettre l'insertion d'une partie de l'élément 5 de liaison. Ce dernier constitue un plot double, formé de deux demi-plots 11, 12, ayant chacun une forme générale en coin et oblongue. Le demi-plot 11 présente ainsi deux grandes faces opposées 11a reliées par deux petites faces 11b et le demi-plot 12 présente également deux grandes faces opposées 12a reliées par deux petites opposées 12b. Ces deux demi-plots 11, 12 sont fixés l'un à l'autre par soudage ou tout autre moyen approprié, de façon à constituer une pièce monobloc, en étant décalés angulairement de 90 degrés l'un par rapport à l'autre.

Sur les grandes faces 12a sont formées des nervures allongées saillantes 14, à raison d'une par face dans cet exemple, et de même sur les milieux des grandes faces opposées 11a sont agencées deux nervures similaires 15.

Dans la face plane 16 de la tête sphérique 1, est agencée une seconde fente allongée 17, similaire à la fente 9. Sur les parois opposées de ces deux fentes 9, 17, est réalisée une série de cannelures 18, 19 conjuguées des nervures correspondantes 15 et 14. Le nombre des cannelures 18, 19 peut varier en fonction de l'amplitude du réglage souhaité pour la position du plot 5 dans les fentes 9, 17.

En effet ces dernières et les demi-plots correspondants 11, 12 sont dimensionnés de façon que les nervures 15 puissent venir coulisser dans les cannelures choisies 18 et que les nervures 14 puissent coulisser dans des cannelures associées 19.

En fonction des couples de cannelures 18 et 19 choisis pour les nervures 15 et 14, le plot 5 peut prendre différentes positions longitudinales dans la fente 9 et dans la fente 17, ces deux fentes étant sensiblement orthogonales lorsque le demi-plot 11 est engagé dans la fente 9 et que la tête 1 coiffe le demi-plot 14. Ainsi la position de la tête humérale 1 peut être réglée dans deux directions pratiquement orthogonales, ce qui permet de l'adapter au mieux à l'anatomie de l'humérus et de la glène 2 sans devoir porter atteinte à celle-ci.

Il convient de noter que la forme en coin de chaque demi-plot 11, 12 facilite son insertion et son blocage dans la fente respective 9, 17.

Le plot 5 présente des faces terminales opposées 21, 22, venant respectivement en regard du fond de la fente 17 de la tête 1 et du fond de la fente 9 de la queue 4. Ces deux faces opposées 21, 22 sont planes, et dans la réalisation des figures 1 à 3, parallèles entre elles. Les demi-plots 11, 12 et les fentes 9, 17 sont de préférence dimensionnées pour que chaque demi-plot puisse être inséré indifféremment dans la fente 9 ou la fente 17.

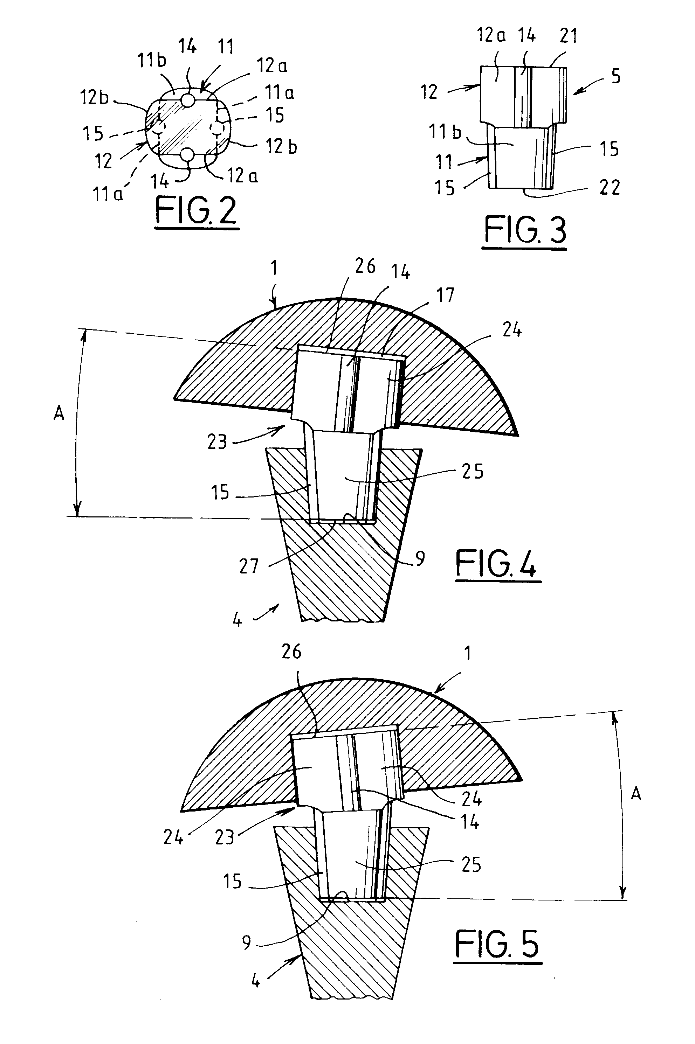

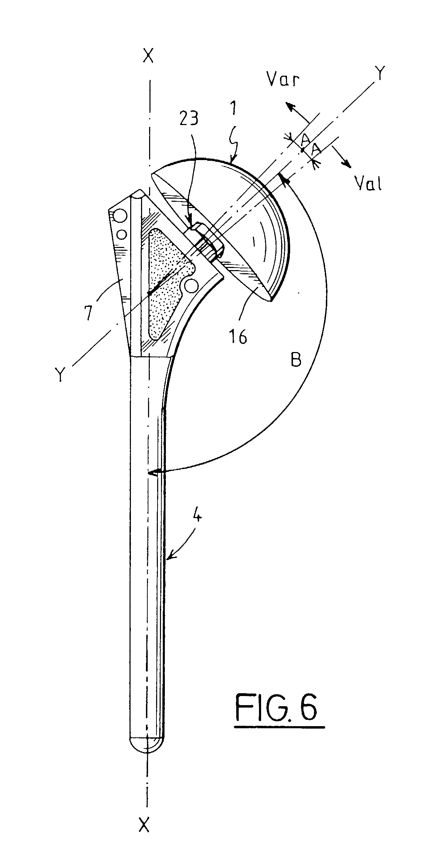

Dans son second mode de réalisation, illustré aux figures 4 à 6, le plot 23 comporte un demi-plot 24 légèrement incliné par rapport au demi-plot 25 associé à la fente 9. Plus précisément, la face 26 du demi-plot 24 est inclinée sur la face opposée 27 du demi-plot 25 d'un angle A, relativement faible, par exemple de 4 à 7 degrés environ et de préférence 6 degrés. Par ailleurs, comme dans la réalisation précédente, les fentes 9, 17 et le plot 23 sont dimensionnés de manière que chaque demi-plot 24, 25 puisse être introduit indifféremment dans l'une ou l'autre fente 9, 17. Il en résulte que des inclinaisons différentes de la tête humérale 1 dans le plan sagittal ou dans le plan frontal peuvent être obtenues en fonction du positionnement de chaque plot 24, 25 dans l'une ou l'autre fente 9, 17.
- Si comme illustré aux figures 4 et 5, le plot 24 est introduit dans la fente 17 de la tête 1, on réalise un inclinaison de celle-ci soit en rétroversion (figure 4) en considérant que la prothèse 1, 4 représentée est celle d'un humérus droit vu depuis l'omoplate, soit en antéversion (figure 5). Ce choix d'orientation de la tête humérale 1 en rétroversion ou en antéversion est possible par simple rotation du plot 23 de 180 degrés autour de l'axe YY.
- Si l'on retourne le plot 23 à partir de sa position représentée à la figure 4, afin d'introduire le demi-plot 24 dans la fente 9 et le demi-plot 25 dans la fente 17, avec rotation de 90 degrés pour que les nervures 15 puissent venir s'introduire dans les cannelures 19 et que les nervures 14 puissent s'introduire dans les cannelures 18, on réalise une inclinaison de la tête 1 sur l'axe YY (figure 6), de l'angle A soit en varus, soit en valgus dans le plan frontal (références Var et Val sur la figure 6). Cette inclinaison, qui peut être de 4 à 7 degrés et de préférence 6 degrés, diminue ou augmente d'autant l'angle B (de préférence 135 degrés).

Ainsi, le plot 23 permet de choisir pour la tête humérale 1 :
- soit une inclinaison dans le plan sagittal en antéversion ou rétroversion,
- soit dans le plan frontal une inclinaison en Varus ou en Valgus.

La possibilité de régler la position de la tête sphérique 1 dans deux directions perpendiculaires grâce aux plots 5 ou 23 et aux fentes cannelées 9, 17, combinées le cas échéant à un réglage supplémentaire de l'antéversion ou de la rétroversion, ou en valgus-varus, confère à la prothèse selon l'invention une très grande adaptabilité à l'anatomie de l'humérus et de la cavité glénoïde 2 du patient. La prothèse assure ainsi une complète reconstruction de l'épaule pratiquement sans incidence sur les muscles et sur l'omoplate, et ce avec une tête 1 unique, mais évidemment de taille variable en fonction du patient. Ainsi est obtenu un avantage appréciable par rapport à certaines réalisations de l'état de la technique antérieure, qui nécessitent un jeu complet de prothèses pour chaque taille de patient.

L'invention n'est pas limitée aux modes de réalisation décrits et peut comporter des variantes d'exécution. Ainsi il est possible de modifier le plot 23 de manière à incliner les faces 26 et 27 relativement l'une à l'autre simultanément dans le plan sagittal et dans le plan frontal, un plot déterminé assurant ainsi une inclinaison déterminée à la fois en antéversion rétroversion et en valgus-varus. Dans ce cas il est possible de réaliser un jeu de plusieurs plots avec des angles variables dans les deux plans frontal et sagittal.

Par ailleurs seule l'une des fentes 9 et 17 pourrait être prévue, soit sur la queue 4, soit sur la tête 1, le plot 5 ou 23 étant fixé à l'élément dépourvu de fente par tout moyen approprié. Le nombre de cannelures 18, 19 peut largement varier en fonction du débattement souhaité, et la liaison entre le plot et la tête 1 ainsi que la queue 4 pourrait être réalisée par tout autre moyen approprié que les nervures 14, 15 et les cannelures 18, 19. La fente 9 est agencée, soit longitudinalement et fermée à ses extrémités (Fig.1), soit transversalement et débouchante à ses extrémités.

Par ailleurs il convient d'observer que chaque demi-plot 11, 12 possède une paire de nervures latérales opposées 15, 14 décalées angulairement d'environ 90 degrés par rapport aux nervures de l'autre demi-plot 14, 15.

## Revendications

1. Prothèse de reconstruction d'épaule, comportant une tête humérale sphérique (1) adaptée pour pouvoir être implantée dans la cavité glénoïde (2) d'une omoplate (3), une queue humérale (4) destinée à être introduite dans le canal médullaire d'un humérus, ainsi qu'un élément de connexion (5 ; 23) entre la tête et la queue, caractérisée en ce qu'une fente (9 ou 17) est agencée dans l'une des deux surfaces (8, 16) en vis-à-vis de la tête et de la queue, en ce que la prothèse est pourvue d'un plot (5; 23) solidaire de celle de ces pièces qui ne présente pas la fente, dans laquelle le plot est adapté pour s'y engager, et en ce qu'il est prévu des moyens (14,15, 18, 19) pour régler la position longitudinale du plot dans la fente (9) et donc la position longitudinale de la tête par rapport à la queue.

2. Prothèse selon la revendication 1 caractérisée en ce qu'elle présente deux fentes (9, 17), sensiblement orthogonales quand la tête (1) et la queue (4) sont assemblées, et en ce que le plot (5) est formé de deux demi-plots (11, 12) fixés l'un à l'autre bout à bout et qui peuvent respectivement s'engager dans une fente correspondante (9, 17), en permettant un réglage de la tête par rapport à la queue dans les deux directions orthogonales définies par les fentes.

3. Prothèse selon la revendication 1 ou 2, caractérisée en ce que la fente (9) dans l'extrémité proximale (7) de la tige (4) est agencée soit longitudinalement et fermée à ses extrémités, soit transversalement et débouchante à ses extrémités.

4. Prothèse selon l'une des revendications 2 et 3, caractérisée en ce que chaque demi-plot (11, 12) est muni de nervures latérales saillantes (15, 14) adaptées pour pouvoir coulisser dans des cannelures conjuguées (18, 19) formées sur les parois opposées des fentes (9, 17), ce qui permet de régler la position longitudinale de chaque demi-plot (11, 12) dans la fente associée (9, 17).

5. Prothèse selon la revendication 4, caractérisée que chaque demi-plot (11, 12) possède une paire de nervures latérales opposées (15, 14) décalées angulairement d'environ 90 degrés par rapport aux nervures de l'autre demi-plot (14, 15).

6. Prothèse selon l'une des revendications 1 à 5, caractérisée en ce que les faces opposées (21, 22) venant du plot (5) respectivement en regard du fond de la fente (9) de la queue (4) et en regard du fond de la fente (17) de la tête (1) sont planes et parallèles (21, 22) entre elles.

7. Prothèse selon l'une des revendications 2 à 6, caractérisée en ce que le plot (5; 23) et les fentes (9, 17) sont dimensionnés de manière que chaque demi-plot (11, 12 ; 24, 25) puisse être introduit indifféremment dans l'une ou l'autre fente.

8. Prothèse selon l'une des revendications 2 à 5, caractérisée en ce que la face (26) de l'un (24) des demi-plots (24, 25) est inclinée sur la face opposée (27) de l'autre demi-plot (25) d'un angle (A) qui, lorsque l'un (24) des demi-plots est introduit dans la fente (17) de la tête, incline celle-ci de l'angle (A) en antéversion ou en rétroversion dans le plan sagittal, et lorsque l'autre demi-plot (25) est introduit dans ladite fente (17) incline la tête en varus ou en valgus dans le plan frontal.

9. Prothèse selon l'une des revendications 2 à 8, caractérisée en ce que les deux demi-plots (11, 12; 24, 25) présentent une forme en coin facilitant l'insertion et le blocage dans les fentes respectives (9, 17).
